# EUROPEAN PATENT APPLICATION

(11) **EP 3 574 927 A2**
(43) Date of publication of application: **04.12.2019**
(21) Application number: 19157934.1
(22) Date of filing: 19.02.2019
(51) Int. Cl.: A61L 2/10, A61F 17/00

(54) **PORTABLE TELEMEDICINE SYSTEM WITH INTEGRATED MEDICAL IMAGING AND UV STERILIZATION**

(30) Priority: 20.02.2018 US 201862632580 P; 06.02.2019 US 201916269288
(71) Applicant: Covidien LP, Mansfield, MA 02048 (US)
(72) Inventor: HAG, Eslam E., Chandler, AZ Arizona 85248 (US)
(74) Representative: Maschio, Antonio

(57) **Abstract**

The present disclosure relates to portable medical systems and methods for deploying a remote surgical theater. The portable medical system may include a portable medical container, a sanitization compartment, and a power supply. The portable medical container may be transitionable between a closed position and an open position. The sanitization compartment may be disposed within the portable medical container and transitionable between a closed position and an open position. The power supply may be disposed within the portable medical container, the power supply configured to selectively supply electrical power to the sanitization compartment. The power supply may be configured to receive user input and, in response to the user input, transmit the electrical power to the sanitization compartment.

## Description

### BACKGROUND

### Technical Field

The present disclosure relates to portable telemedicine and telemedical systems and, more particularly, to deployable system for creating and sustaining a portable surgical theater in remote locations.

### Description of Related Art

Systems and methods have been developed and are known in the art to provide therapeutic medical treatments to patients who are not in a controlled surgical environment such as hospitals or clinician offices. For example, portable containers with medical supplies, commonly referred to as first aid kits, are often used by clinicians such as doctors and paramedics to transport equipment useful for delivering medical care to patients who are otherwise unable to travel to a medical facility. These first aid kits may include a variety of tools, such as prepackaged sterilized equipment and medicines.
Additionally, systems for providing specific therapeutic treatments are often enclosed in stand-alone kits, such as defibrillators, intubation kits, and vital sign measurement kits ("stand-alone kits"). These stand-alone kits are designed to maintain sterility prior to use by clinicians, and are generally directed to providing life-sustaining support to a patient prior to transportation of the patient to a medical facility. While these tools and methods are sufficient for short-term medical treatment of patients (e.g., stabilization of the patient), there exists a need for systems and methods that facilitate prolonged remote therapeutic treatment to patients.

### SUMMARY

In accordance with an aspect of the present disclosure, a portable medical system for deploying a remote surgical theater includes a portable medical container, a sanitization compartment, and a power supply. The portable medical container is transitionable between a closed position and an open position. The sanitization compartment is disposed within the portable medical container and transitionable between a closed position and an open position. The power supply is disposed within the portable medical container and is configured to selectively supply electrical power to the sanitization compartment. The power supply is configured to receive user input and, in response to the user input, transmit the electrical power to the sanitization compartment.

In aspects, the sanitization compartment may be configured to transmit UV light in response to receiving the electrical power. The sanitization compartment may be configured to release a sanitization agent including ozone gas, heated air, ethylene oxide, microwave-energy, hydrogen peroxide vapor, hydrogen peroxide plasma, and/or formaldehyde gas. The sterilization compartment may be removably disposed within the portable medical container.

According to aspects, the portable medical container may further include a compartment selected from the group consisting of a fluid delivery compartment, an electrosurgical generator, a removable power supply, and an air recirculation system compartment. The compartment may be removable.

In aspects, the portable medical system may further include a shroud having at least one opening configured to have an air connection hose coupled thereto to facilitate air circulation thereacross. The portable medical system may include a console configured to be in electrical communication with the power supply and/or a remote console. The portable medical system may include at least one medical device selected from the group consisting of an imaging device, an electrically powered surgical instrument, and an electrosurgical instrument.

According to aspects, the power supply may be configured to receive electrical power from an external power source and, in response to receiving the electrical power, store the electrical power in one or more batteries disposed in the power supply. The console may be configured to establish wireless communication with a remote console.

In aspects, when wireless communication is established between the console and the remote console, the console is configured to receive user input at the console, transmit the user input to the remote console, and in response to transmitting the user input, receive second user input at the remote console. When wireless communication is established, the console may further be configured to display the second user input on a display of the console.

In accordance with an aspect of the present disclosure, a method of monitoring a telemedical procedure includes deploying a console at a remote surgical theater located in a first location, establishing electrical communication with a second console in a second location, inputting user input into the console at the first location to be transmitted to a clinician interacting with the second console at the second location, transmitting the user input from the console at the first location to the console at the second location, receiving at the console at the first location, in response to transmitting the user input, second user input received at the second console, and displaying the second user input on a display of the console at the first location.

In aspects, the method may further include compressing the user input received at the console at the first location prior to the transmitting. The method may further include compressing the second user input received at the second console prior to the receiving. The method may further include receiving, at the console at the first location, instructions to control a surgical device in electrical communication with the console, and transmitting control signals from the console based on the instructions to control the surgical device.

### BRIEF DESCRIPTION OF THE DRAWINGS

The accompanying drawings, which are incorporated in and constitute a part of the specification, illustrate embodiments of the disclosure and, together with a general description of the disclosure given above, and the detailed description of the embodiment given below, serve to explain the principles of the disclosure.
FIG. 1 is a perspective view of a first portable telemedicine kit in a closed position, in accordance with an embodiment of the present disclosure;
FIG. 2 is a plan view of the first portable telemedicine kit of FIG. 1 in an open position;
FIG. 3 is a plan view of an alternate embodiment of the portable telemedicine kit of FIG. 1, including a first and second portable telemedicine kit, in accordance with an embodiment of the present disclosure;
FIG. 4 is a perspective view of a surgical theater having the portable telemedicine kit of FIG. 1 disposed therein, in accordance with an embodiment of the present disclosure;
FIG. 5 is a flow diagram illustrating electrical communication between a console of the portable telemedicine kit of FIG. 1 and one or more remote consoles; and
FIG. 6 is a schematic block diagram of a computing device that may be employed according to various embodiments of this disclosure.

### DETAILED DESCRIPTION

Systems which enable the construction of a portable medical environment, as disclosed herein, may be housed in one or more transportable containers. More particularly, these portable medical systems include equipment for sustaining a surgical theater in a location remote from an established medical facility. The portable medical systems may contain equipment found in hospitals, in-patient or out-patient medical facilities, and the like. The equipment included in the portable medical systems may be permanently integrated or removably coupled to individual portable containers. Alternatively, the equipment of any portable medical system may be distributed across multiple portable containers as a distributed the portable medical system and increase the ease of transport of the portable medical system.

Equipment included in portable medical systems may include one or more therapeutic treatment systems such as, without limitation, electrosurgical generators, airway intubation kits, surgical tools and accessories, and the like. These therapeutic treatment systems are housed within one or more containers, such as soft-side containers ("bags") or hard containers such as hard plastic containers and metal containers. In embodiments, multiple containers may be operably interconnected to allow for the distribution and interconnection of therapeutic treatment systems. As will be discussed later in detail, the therapeutic treatment systems include components such as, without limitation, a surgical shroud, an air filtration and recirculation system, an ultra-violet sterilization system, and a telecommunication system. Distribution of these components may permit a clinician to disperse the weight and size of each of the portable containers of the various portable medical systems, which in turn may enable the described portable medical systems to be deployed to remote locations which otherwise do not have access to permanent medical facilities.

Embodiments of the present disclosure are described in detail with reference to the drawings, in which like reference numerals designate identical or corresponding elements across the several included views. As defined herein, the term "clinician" refers to a doctor, nurse, or other care provider and may include support personnel. The term "distal" refers to structure that is farther from a clinician, while the term "proximal" refers to structure that is closer to the clinician. Further, directional terms such as front, rear, upper, lower, top, bottom, distal, proximal, and the like are used to assist in understanding the description and are not intended to limit the present disclosure.

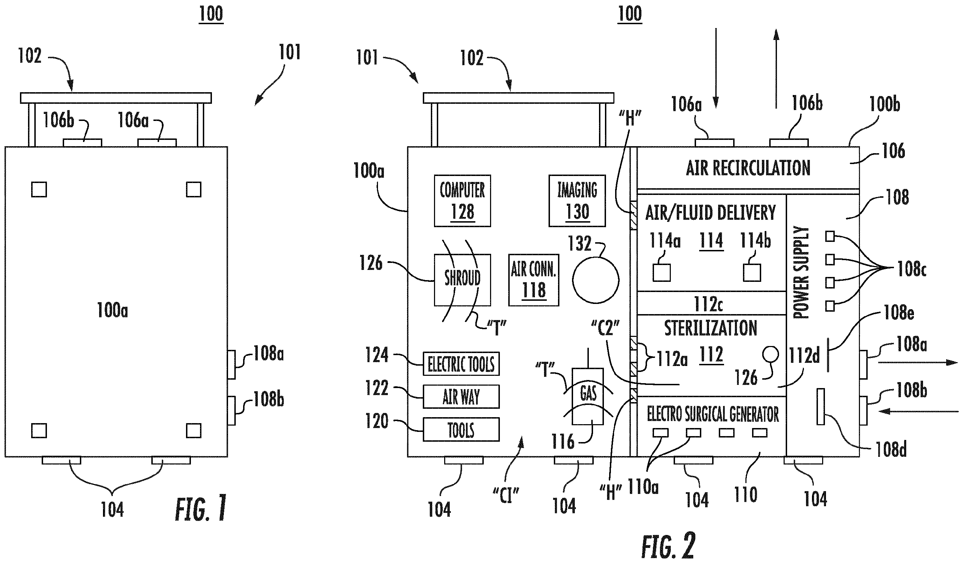

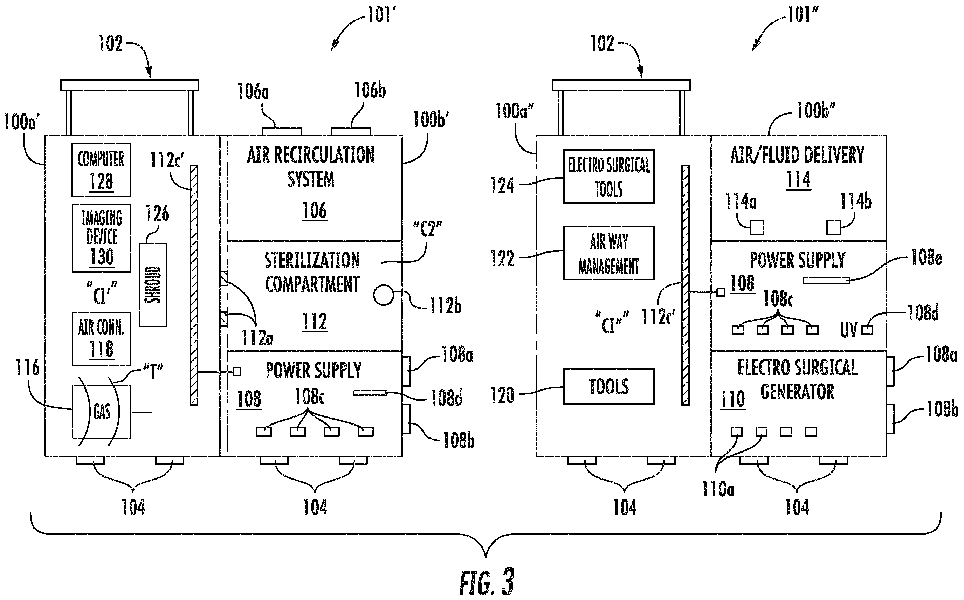

As will be described in detail, the provided descriptions illustrate embodiments of portable medical systems for selectively deploying a portable medical environment, the portable medical systems designated generally 100. The portable medical system 100 may be contained in a single portable medical container 101 (FIG. 1), or distributed across multiple portable medical containers 101', 101" (FIG. 3). The portable medical containers 101, 101', 101" (FIGS. 1-3) may include multiple interchangeable compartments (i.e., a sterilization compartment 112). In embodiments, certain interchangeable compartments may be fixed or otherwise permanently affixed to and integrated with the portable medical containers 101, 101', 101". Additionally, the portable medical container 101 includes at least one compartment "C1" (FIG. 2) dimensioned to store components such as, without limitation, a surgical shroud or shroud 126 and surgical tools 120 (*see* FIG. 1). The portable medical systems 100 may further include an air recirculation system 106, a power supply 108, an electrosurgical generator 110, a sterilization compartment 112, and a gas or fluid delivery system 114. It is contemplated that the portable medical system 100 of the present disclosure may, in embodiments, contain any subset of components or containers thereof.

Referring initially to FIG. 1, a portable medical system 100 includes a portable medical container 101. The portable medical container 101 defines a housing having a first half portion 100a and a second half portion 100b. The first half portion 100a and the second half portion 100b are rotatably coupled to one another about a hinge "H". The hinge "H" is configured to permit rotational movement of the first half portion 100a and the second half portion 100b between a closed position (FIG. 1) and an open position (*see* FIG. 2). The hinge "H" may include one or more removable pins (not shown) which permit selective detachment and reattachment of the first half portion 100a and the second half portion 100b. When the pins are removed, the first and second half portions 100a, 100b may be separated and positioned about a remote surgical theater 200 to facilitate clinician engagement with the components therein during surgical procedures. It is contemplated that the first and second half portions 100a, 100b may be coupled in any suitable manner (e.g., via clips, hinges, and the like).

The first and second half portions 100a, 100b further include a plurality of housing supports 104 extending from an exterior surface of the first and second half portions 100a, 100b. The housing supports 104 may be coupled to an inner frame (not shown) enclosed by the first and second half portions 100a, 100b. When the portable medical system 100 is placed on a surface (e.g., the ground or along a table), the housing supports 104 distribute forces received by the first and second half portion 100a, 100b across the inner frame. Distribution of the forces associated with the weight of the portable medical system 100 along the internal frame maintains stability of the portable medical system 100 during surgical procedures and transportation. Distribution of the forces additionally reduces the chance for unintended application of force to the components of the portable medical system 100, which may cause harm to the components disposed therein.

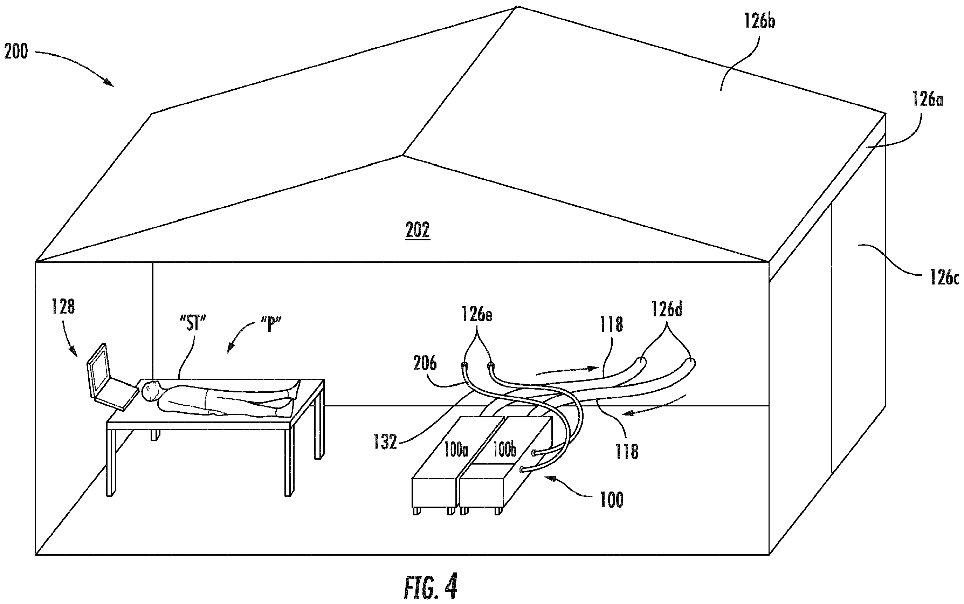

With continued reference to FIG. 1, the portable medical system 100 includes an air intake port 106a, an air exhaust port 106b, a power input port 108a, and a power output port 108b. The air intake port 106a and air exhaust port 106b are coupled to, and are in fluid communication with, an air recirculation system 106 (FIG. 2). The air recirculation system 106, which is used to filter air within a surgical space 202 (FIG. 4), exchanges air within the surgical space 202 with air outside the surgical space (either in an air canister or air located directly outside the surgical space 202), or both. The air recirculation system 106 may be any suitable air propulsion system having one or more fans disposed therein. The air recirculation system 106 is enclosed within the second half portion 100b of the portable medical system 100. In embodiments, the air recirculation system 106 filters air prior to introduction to the surgical space 202. The air intake port 106a and air exhaust port 106b extend through the portable medical container 101, enabling ingress and egress of air to and from the air recirculation system 106. The air intake port 106A and air exhaust port 106B may further include filters, in addition to a filtration system enclosed in the air recirculation system 106, disposed therein capable of filtering particulates and air contaminants as air passes therethrough. The air intake port 106a and air exhaust port 106b may optionally be coupled to fluid caps (not shown) which prevent ingress or egress of air into or out of the air recirculation system 106, or contamination of the air recirculation system 106 during transportation of the portable medical system 100.

The portable medical system 100 includes a power supply 108 having a power input port 108a and power output port 108b (FIG. 2). The power input port 108a and the power output port 108b extend through the portable medical container 101 and enable external coupling of electrical cables and devices for electrically charging or distributing of electric power from the power supply 108. Specifically, the power input port 108a is configured to receive either 110V or 220V connections to permit recharging of one or more batteries (not shown) located within the power supply 108. In embodiments, the power input port 108a may be any suitable port capable of receiving electrical power (e.g., alternating current (AC) or direct current (DC) electrical power) to charge the one or more batteries of the power supply 108, or power one or more devices coupled thereto. For example, one or more solar collectors (or solar panels) may be electrically coupled to the power input port 108a and provide electrical power, particularly when the portable medical system 100 is disposed in an environment where power connections are unavailable. Likewise, the power output port 108b is configured to receive 110V or 220V connections, and permit transmission of electrical power to instruments which require electrical power for operation. Similar to the air intake port 106a and air exhaust port 106b, the power input port 108a and power output port 108b may include fluid caps (not shown) which prevent ingress of debris or fluids during transportation or operation of the portable medical system 100.

The power supply 108 includes multiple receptacles 108c configured to receive and deliver power to electrical plugs connected thereto. The receptacles 108c are located within the portable medical container 101 and are accessible when the first and second half portions 100a, 100b of the portable medical container 101 are in the open configuration (FIG. 2). During operation, the power output port 108b and the receptacles 108c may be selective powered by the power supply 108. For example, the power supply 108 may supply power to the power output port 108b when the portable medical system 100 is in the closed configuration and alternatively may supply power to the receptacles 108c when the portable medical system 100 is in the open configuration. Alternatively, electrical power may be selectively transmitted to the power output port 108b or the receptacles 108c based on user input (e.g., via a switch (not shown)). An electrical charge indicator 108e is located along the power supply 108 and shows the battery state of charge.

An electrosurgical generator 110 is enclosed within the second half portion 100b of the portable medical system 100. The electrosurgical generator 110 includes ports 110a which are configured to selectively receive electrosurgical instrument connectors (not shown) electrically coupled to one or more electrosurgical instruments 124. For example, the electrosurgical generator 110 may be configured transmit electrical power to, without limitation, handheld devices such as monopolar energy delivery devices, bipolar energy delivery devices, surgical staplers configured to engage and seal vessels, and the like. Examples include, without limitation, the surgical devices described in U.S. Patent No. 9,375,282, as well as U.S. Patent No. 9,375,258, the entire contents of both of which are hereby incorporated by reference in their entirety. The electrosurgical generator 110 is configured to transmit electrosurgical energy for use by electrosurgical instruments 124 capable of transmitting either microwave energy or electrosurgical energy. The electrosurgical generator 110 is further configured to receive a return electrical current from the electrosurgical instruments 124 or electrosurgical instrument accessories (e.g., a return electrode or pad). The electrosurgical generator 110 is electrically coupled to the power supply 108, thereby permitting electrical communication between the power supply 108 and the electrosurgical generator 110. The electrosurgical generator 110 may be configured to be in either wired or wireless electrical communication with a computing device or console 128 and to transmit relevant information to the console 128 during a surgical procedure, e.g., the operational state, temperature, or life cycle information of the electrosurgical generator 110. Electrosurgical generators 110 suitable for inclusion may include Medtronic's Valleylab™ LS10 Generator, or other similar electrosurgical generators.

A sterilization compartment 112 is positioned within the portable medical container 101 and is in electrical communication with the power supply 108. The sterilization compartment 112 includes a door 112d pivotably coupled to the second half portion 100b of the portable medical system 100 via hinges 112a. It is contemplated that the door 112d, along with the compartment doors described in the present application, may incorporate any suitable opening and closing mechanism. The door 112d is configured to enclose one or more surgical tools 120 or, more generally, objects disposed in a sterilization compartment "C2" enclosed by the door 112d. The door 112d includes a lock 112b which, when transitioned to a lock position, is configured to secure the door in a closed position relative to the sterilization compartment 112. The lock 112b may be in electrical communication with the power supply 108. When the lock 112b is transitioned by a clinician from the unlock position to the lock position, the power supply 108 transmits electrical power to a UV light bulb located within the sterilization compartment "C2". The UV light bulb may transmit UV light at a preferred wavelength within the UV light spectrum (between approximately 400-180nm, including UV-A (400-320nm), UV-B (320-280nm), and UV-C (280-180nm)), to sterilize medical objects located in the sterilization compartment "C2". It is contemplated that the sterilization compartment 112 may further include other sterilization units configured to disseminate objects disposed in the sterilization compartment "C2" such as, without limitation, an ozone generator, an air heating element, a gas dispersal device configured to disperse ethylene oxide, hydrogen peroxide vapor, hydrogen peroxide plasma, formaldehyde gas, and the like. Additionally, a microwave generator may be included to disperse microwaves into the compartment "C2" to disinfect objects disposed therein.

Additionally, the portable medical system 100 includes a UV light bulb 112c. The UV light bulb 112c, similar to UV light bulb 112c included in the cavity "C1" of the portable medical system 100' (FIG. 3), may deliver any suitable UV light within the UV light spectrum for disinfecting the contents enclosed in the sterilization compartment 112. The UV light bulb 112c is in electrical communication with the power supply 108. The power supply 108 includes a switch 108d which is configured to cause the power supply 108 to transmit electrical power to the UV light bulb 112c. In embodiments, the power supply 108 may periodically transmit electrical power to cause the UV light bulb 112c to emit light to disinfect the contents of the sterilization compartment 112. Also, in embodiments, the power supply 108 may transmit electrical power when one or more predetermined events occur. For example, the sterilization compartment 112 may be locked by a user after one or more surgical tools 120 are placed therein. Once locked, the power supply 108 may receive a sensor signal indicating the closure, and in response, may transmit power to the UV light bulb 112c to disinfect the contents of the sterilization compartment. Transmission of electrical power by the power supply 108 to the UV light bulb 112c may be delayed by the power supply 108 to allow clinicians to close the portable medical container 101. Alternatively, the power supply 108 may intermittently (e.g., at a predetermined period or time interval) cause electrical power to be transmitted by the power supply 108 to the UV light bulb 112c. Transmission of UV light by the UV light bulb 112c may provide concentrated or prolonged sterilization of the contents located within the compartment "C1" of the portable medical container 101. For example, if certain components of the portable medical system 100 come into contact with contaminants such as, without limitation, the shroud 126, and are not sterilized in the sterilization compartment 112, during transportation or storage the power supply 108 may power the UV light bulb 112c to sterilize such components.

A fluid delivery system 114 is located within the portable medical container 101 and is in electrical communication with the power supply 108. The fluid delivery system may be in wired or wireless communication with the console 128. The fluid delivery system includes an input port 114a and an output port 114b which are configured to receive and transmit fluids. More particularly, the fluid delivery system 114 may receive either liquids or gases during a surgical procedure. The liquids or gases may be distributed from one or more containers coupled to the fluid delivery system 114, such as a gas container 116 or an intravenous (IV) bag (not shown). The fluid delivery system 114, when connected to the gas container 116 or IV bag, may distribute the contents of the respective container to a patient to reduce pain, transition the patient into a partially or completely sedated state, or therapeutically treat the patient during surgical procedures. The fluid delivery system 114 may receive control signals from the computer or console 128 to regulate the flow of fluid to and from a patient.

With continued reference to FIG. 2, the second half portion 100b defines a cavity or compartment "C1" and includes a gas container 116, air recirculation piping or hoses 118, one or more surgical tools 120, an airway intubation kit 122, electrosurgical instruments 124, a tent or shroud 126, the console 128, and a portable imaging device 130. The first compartment "C1" is configured to receive items such as the gas container 116 or the portable imaging device 130 which are removable from the portable medical system 100, and may include one or more tie-downs "T". The tie-downs "T" may be constructed of rigid materials such as plastics or malleable materials such as flexible cord. When secured by the tie-downs "T" the contents located in the first compartment "C1" are prevented from shifting during transit, potentially leading to damage of the contents disposed within the first compartment "C1".

The gas container 116 may include gasses to be delivered to patients to transition the patients to sedated states during surgical procedures. Additionally, the gas container 116 may include air with a heightened oxygen concentration or other gas mixtures for therapeutically treating patients. The airway intubation kit 122 may include a tracheostomy tube, a scalpel, one or more syringes, and other tools known in the art for establishing an airway in a patient. The electrosurgical instruments 124 may include, without limitation, electrosurgical pencils and forceps, monopolar or bipolar sealers, dissection devices such as harmonic scalpels, probes for electrosurgical ablation, probes for microwave ablation, and other similar electrosurgical instruments known in the art.

The shroud 126 is configured to enclose a remote surgical theater 200 when deployed (FIG. 4). The shroud 126 includes a frame 126a and a membrane 126b. The frame 126a is configured to support the membrane 126b thereon and to provide a semi-permeable or impermeable barrier between a surgical space 202 and the surrounding environment of the remote surgical theater 200. The membrane 126b includes entry flaps or doors 126c which enable ingress and egress of clinicians and patients through the membrane 126b. The membrane 126b includes a pair of fluid openings 126d disposed along the surface of the membrane 126b. The pair of fluid openings 126d may be operably connected to the air intake port 106a air exhaust port 106b. Likewise, the membrane 126b includes a pair of electrical connection openings 126e which may be operably connected to the power input port 108a and power output port 108b.

Air connection hoses or hoses 118 are configured to establish a fluid connection between the air intake port 106a or air exhaust port 106b of the air recirculation system 106 and the fluid openings 126d of the membrane 126b. In use, the hoses 118 permit the exchange and recirculation of air within a surgical space 202 formed when the shroud 126 is deployed. Likewise, electrical power cables 132 are configured to operably couple the power supply 108 to an external power source (not shown). More particularly, when the remote surgical theater 200 (FIG. 4) is within a predetermined distance from an external electrical power source (e.g., a portable power generator or an electrical outlet) the electrical power cables 132 may be coupled to both the external power source and the power input port 108a of the power supply 108.

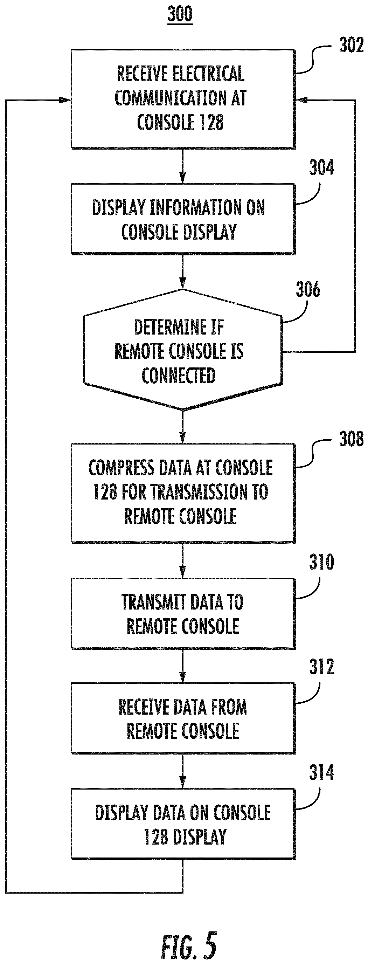

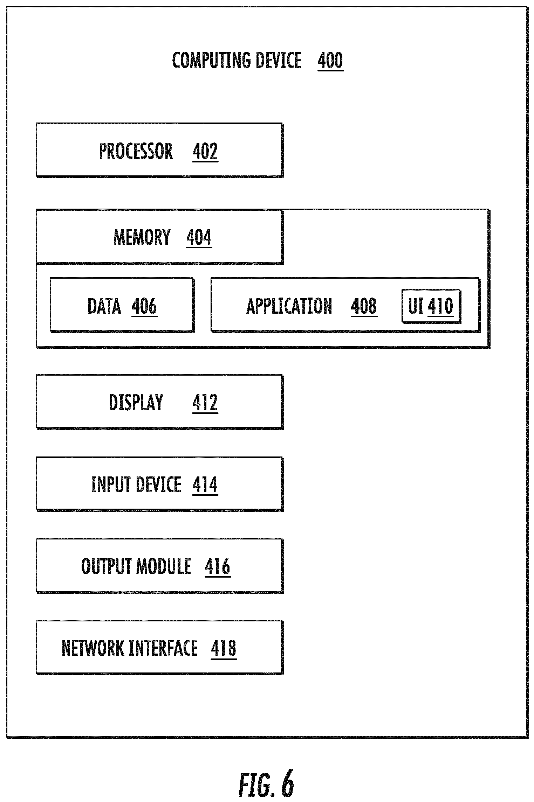

The console 128 may be any suitable computing device, such as the computing device 400 of FIG. 6) capable of, either via a wired or wireless connection, electrically communicating with the power supply 108, the electrosurgical generator 110, the portable imaging device 130, or any other device included in the portable medical system 100. The console 128 includes a communications interface or antenna capable of establishing a data connection either via wireless configurations, e.g., Wi-Fi®, Bluetooth, LTE, and/or wired configurations. Additionally, or alternatively, the communications interface may be capable of establishing wired or wireless connections with internet service providers via a wired or wireless configuration. A detailed description of communication between the console 128 and one or more remote consoles (not shown) will be described with reference to FIG. 5.

The portable imaging device 130 may be any portable imaging device such as a portable ultrasound device or a portable x-ray device, capable of being housed within the compartment "C1" of the portable medical system 100. The portable imaging device 130 may further be configured electrically communicate with the console 128, either via a wired or wireless connection. When the console 128 is in electrical communication with a remote console, the console 128 may operably connect the portable imaging device 130 with the remote console, enabling the transmission of data collected during use of the portable imaging device 130 by clinicians in the remote surgical theater 200 (FIG. 4) to remote clinicians. As will be discussed later, data received from the portable imaging device 130 by the console 128 may be compressed prior to transmission to the remote console. In embodiments, the portable imaging device 130 may be included separately from the portable medical system 100, or may be any suitable portable imaging device configured to electrically communicate with the console 128.

It is contemplated that the portable medical system 100 may further include optional medical equipment (not shown) such as, without limitation, dialysis devices, additional or independent communications devices, electrocardiogram (ECG / EKG) monitors, electroencephalography machines, blood pressure monitoring devices, pulse oximeters, and the like. Further, disposable instruments such as electrodes, gloves, forceps, and the like may be included in the portable medical system 100. An example of suitable medical equipment for inclusion in the portable medical system 100 includes portable therapy deliver devices described in commonly-owned U.S. Patent Application Publication No. 2006/0270916, entitled "PORTABLE THERAPY DELIVERY DEVICE WITH A REMOVABLE CONNECTOR BOARD" the entire contents of which are hereby incorporated by reference.

Adapters (not shown) may be included in the portable medical system 100 to facilitate interconnection between components included in the portable medical system 100 and surgical equipment available at a remote surgical theater 200 (*see* FIG. 4). For example, in addition to or in place of the portable imaging device 130, the console 128 may be coupled to imaging devices available at remote locations (e.g., a magnetic resonance imaging (MRI) machine or an x-ray machine) via an adapter (not shown). Likewise, electrosurgical generators, or other surgical equipment available at the remote surgical theater 200 may be coupled to the console 128 or the power supply 108 via adapters to enable the portable medical system 100 to engage and/or power the available surgical equipment.

When connected to surgical equipment available at the remote surgical theater 200, the console 128 may receive data from the surgical equipment and display the data determined to be relevant to the surgical procedure being performed. Additionally, the surgical equipment, when in electrical communication with the console 128, may be operably coupled to remote consoles (not shown), enabling remote clinicians to analyze data received from the remote surgical equipment. For example, during a telemedical procedure, clinicians at remote surgical theaters 200 may couple a portable X-ray scanner to the console 128 to collect one or more X-ray images of a patient. The console 128 may subsequently transmit the images, periodically or in real time, to clinicians located remotely for analysis. Analysis performed by the remote clinicians may be incorporated into feedback which is transmitted back to the console 128 to assist the clinicians during the surgical procedure (*see* FIG. 5).

Referring to FIG. 3, illustrated is an alternative embodiment of the portable medical system 100, in which the components of the portable medical system 100 are distributed across two portable medical containers 100a', 100a". By distributing the components across the two portable medical containers 100a', 100a" the overall weight of the portable medical system 100 is dispersed. It is contemplated that, depending on the components included in the portable medical system 100, when the collective weight of the portable medical system 100 reaches an undesirable weight (e.g., fifty pounds) or more the two portable medical containers 100a', 100a" may be transported to the remote surgical theater 200 (FIG. 4) as a distributed portable medical system 100'. When distributed across two portable medical containers 100a', 100a", each container 100a', 100a" may include either air intake port 106a and air exhaust port 106b, power input port 108a and power output port 108b, or any combination of ports thereof. Depending on the available input and output ports located along each container clinicians may selectively include the air recirculation system 106 and the power supply 108 within the portable medical containers 101', 101".

FIG. 4 illustrates a remote surgical theater 200. To deploy the portable medical system 100, 100', a clinician assembles the frame 126a of the shroud 126 to form an assembled frame 126a. Once the frame 126a is assembled the membrane 126b is placed or draped over the frame 126a. If an existing structure is available at the remote surgical theater 200, assembly of the frame 126a may not be necessary, and the membrane 126b may be draped over the existing structure. Draping the membrane 126b over the frame 126a encloses a surgical space 202 which is shielded from weather or distractions including, without limitation, rain, sunlight, and sound.

The surgical space 202 includes a surgical table "ST" on which a patient "P" may be disposed. During surgical procedures, clinicians may interact with the console 128. The console 128 may have a camera and microphone disposed thereon (not explicitly shown) to collect audio and video data. Upon collection, the console 128 may transmit the received data to one or more remote consoles. In embodiments, the console 128 may compress the data prior to transmission. At the remote console, a clinician may view the surgical procedure, and instruct the clinician during the surgical procedure when desired. Likewise, the clinician at the remote console may monitor the state of the patient "P" and indicate to the clinician performing the surgical procedure the state of the patient "P". For example, if the clinician at the remote console notes that the blood pressure of the patient "P" has changed and that it might be desirable to address the change in blood pressure, the clinician at the remote console may indicate such to the clinician performing the surgical procedure. In embodiments, the clinician at the remote console may supply user input to the remote console 128 to control one or more components in electrical communication with the console 128 disposed in the surgical space 202 such as, without limitation, the electrosurgical generator 110, the air recirculation system 106, and the like.

The membrane 126b may be formed from any semi-permeable or non-permeable material and may be transparent, translucent, or opaque, depending on the level of privacy desired. The membrane 126b forms a pair of doors 126c to permit ingress or egress of clinicians and/or patients into and out of the surgical space 202. The membrane 126b further includes a first pair of fluid openings 126d and a second pair of electrical connection openings 126e. The first pair of fluid openings 126d and the second pair of electrical connection openings 126e are disposed along a surface of the membrane 126b. The first pair of fluid openings 126d are configured to have the hoses 118 coupled thereto to permit communication of air outside the surgical space 202 to and from the air recirculation system 106. Similarly, the second pair of electrical connection openings 126e are configured to permit a pair of electrical power cables 132 to pass through the membrane 126b, enabling external coupling of the power supply 108 to external power sources. Where no external power sources are available, the second pair of electrical connection openings 126e may be sealed by coupling a cap (not shown) to the openings so as to prevent ingress or egress of fluid or contaminants into and out of the surgical space 202.

Referring now to FIG. 5, during remote surgical procedures, communication between the console 128 (FIG. 4) and one or more remote consoles (e.g., a console at an office of a clinician; not shown) may occur, the communication process generally referred to as process 300. While described herein as communication between two consoles 128, one located in a remote surgical theater 200 (FIG. 4) and one located in a remote medical facility (not shown), the present disclosure contemplates communication between the console 128 located at the remote surgical theater 200 and any other suitable computing device capable of receiving and transmitting data in real time between the clinician engaging the console 128 and one or more remote clinicians assisting during the procedure.

Initially, a clinician may position the console 128 within the surgical space 202 in a desirable location for easy access during a surgical procedure. The console 128 may be battery operated or may electrically couple to the power supply 108 located in the portable medical container 101. Electrical communication may be established between the console 128 and the one or more devices included in the portable medical system 100. For example, the console 128 may wirelessly communicate with the electrosurgical generator 110 to provide information regarding operation of the electrosurgical generator 110 during the surgical procedure. As the console 128 receives data from the device or devices in electrical communication with the console 128 (S302), relevant information is displayed via a display of the console 128 or transmitted as audio output to the clinician from the console 128 (S304).

During the surgical procedure, the console 128 determines whether communication has been established with a remote console (S306). More particularly, the console 128 determines if communication has been established between the console 128 located in the remote surgical theater 200, and the remote console 128 (not shown) where the remote clinician is located. If communication is not established, the console 128 continues to receive communications (S302) and display the relevant data to the clinician during the surgical procedure, while optionally indicating that communication has not been established with the remote console 128 (not shown). (S304). Alternatively, once the console 128 determines that communication has been established with a remote console (S306) the console 128 in the remote surgical theater 200 compresses the data received during the surgical procedure in anticipation of transmission to the remote console 128 (S308). The console 128 in the remote surgical theater 200 then transmits the compressed data to the remote console, the data transmitted to enable the remote clinician to assist the acting clinician during the procedure (S310). The console 128 may also encrypt the data prior to transmission to the remote console 128.

As the clinician at the remote console 128 reviews the information received during the procedure (e.g., a video feed of the surgical procedure, one or more measurements taken from a surgical device such as an imaging device, and the like) the clinician at the remote console 128 may input feedback to be transmitted to the clinician performing the procedure. For example, in the case of electroporation, the clinician performing the procedure may navigate an electrosurgical instrument 124 to a target site in a patient. During such navigation, the remote clinician may indicate how to more precisely navigate through the patient, where to apply electrosurgical energy, how much electrosurgical energy to apply to a particular target site, etc. Similar to the compression of the data transmitted to the remote console 128, the data associated with the input of information by the clinician at the remote site may be compressed prior to transmission to the console 128 in the remote surgical theater 200 (S312). When the console 128 receives data from the remote console, the data is displayed on the console 128 (via one or more display units or screens) or output via an audio output device integrated in the console 128 (S314). This process 300 is repeated until the clinician disables communication between the console 128 and the remote console.

In embodiments, process 300 may be interrupted at any point to verify that electrical communication between the console 128 in the remote surgical theater 200 and the remote console 128 is maintained. If the electrical communication is interrupted, process 300 may leave any particular step and repeat by re-executing the process 300. It is contemplated that, in embodiments, the console 128 may receive input solely from a patient (without the aid of a clinician at the console 128), thereby enabling communication with the patient and the remote clinician at the remote console 128. Further, in embodiments, the console 128 may receive sensor signals from the devices during a particular surgical procedure, the devices disposed in the remote surgical theater and in either wired or wireless electrical communication with the console 128. In response to receiving the sensor signals, remote console 128 may execute one or more sets of instructions which, when executed, process the received sensor signals. The instructions may generate images associated with the sensor signals for display on the console 128. More particularly, the generated images may be overlaid onto the images received from the remote console for display on the console 128, or adjacent to the images received by the remote console. For example, as biometric measurements, photos, and/or videos are captured and transmitted as sensor date to the console 128, the sensor data may be processed and used to generate images or video indicative of the data collected to be displayed. The generated images or video may then be overlaid onto the video being displayed by the console 128 during the surgical procedure.

In embodiments, the console 128 is configured to receive sensor signals from one or more portable imaging devices 130 and, based on the sensor signals, generate an image or series of images (video, etc.) to be displayed on the console 128. It is contemplated that, in embodiments.

Referring now to FIG. 6, illustrated is a schematic block diagram of a computing device 400 that may be employed according to various embodiments of this disclosure. Though not explicitly shown in corresponding figures of this application, the computing device 400, or one or more components thereof, may represent one or more components (e.g., console 128, or a control system associated with the air recirculation system 106, the power supply 108, the electrosurgical generator 110, the sterilization compartment 112, the fluid delivery system 114, and/or any of the components included in the cavity "C1" of the portable medical system 100. The computing device 400 may include one or more memories 404, processors 402 in electrical communication with the memories 404, display devices or displays 412, network interfaces 418, input devices 414, and/or output modules 416, or any desired subset of components thereof. The memory 404 may include non-transitory computer-readable storage media for storing data and/or software which include instructions to be executed on the one or more processors 402 and which, when executed, control operation of the computing device 400. In embodiments, the memory 404 may include one or more solid-state storage devices such as flash memory chips. Additionally, or alternatively, the memory 404 may include one or more mass storage devices connected to the processor 402 through a mass storage controller and a communications bus (not shown). Although the description of computer readable media described in this disclosure refers to a solid-state storage device, it will be appreciated by one of ordinary skill that computer-readable storage media may include any available media that can be accessed by the processor 402. More particularly, computer readable storage media may include non-transitory, volatile, non-volatile, removable, non-removable media, and the like, implemented in any method or technology for storage of information such as computer readable instructions, data structures, program modules, or other suitable data access and management systems. Examples of computer-readable storage media include RAM, ROM, EPROM, EEPROM, flash memory, or other known solid state memory technology, CD-ROM, DVD, Blu-Ray or other such optical storage, magnetic cassettes, magnetic tape, magnetic desk storage or other magnetic storage devices, or any other medium which may be used to store information and which can be accessed by computing device 400.

In embodiments, the memory 404 stores data 406 and/or one or more applications 408. The applications 408 may include instructions which are executed on the one or more processors 402 of the computing device 400. In aspects, the application 408 may include instructions which cause a user interface controller 410 to control the display 412 such that a user interface controller 410 is displayed (e.g., a graphical user interface (GUI)). The network interface 418, in certain embodiments, is configured to couple the computing device 400 and/or individual components thereof to a network, such as a wired network, a wireless network, a local area network (LAN), a wide area network (WAN), a wireless mobile network, a Bluetooth® network, the Internet, and the like. The input device 414 may be any device a user may engage to enter input. For example, the input device 414 may include a mouse, keyboard, touch-capacitive display, voice interface, and/or the like. The output module 416 may, in embodiments, include any connectivity port or bus, such as, for example, a parallel port, a serial port, a universal serial bus (USB), or any other similar connectivity port known in the art.

It will be understood that various modifications may be made to the embodiments of the presently disclosed portable medical system 100. Therefore, the above description should not be construed as limiting, but merely as exemplifications of embodiments. Those skilled in the art will envision other modifications within the scope and spirit of the present disclosure.

The invention may be described by reference to the following numbered paragraphs:-
1. A portable medical system for deploying a remote surgical theater, the portable medical system comprising:
   a portable medical container transitionable between a closed position and an open position;
   a sanitization compartment disposed within the portable medical container and transitionable between a closed position and an open position; and
   a power supply disposed within the portable medical container, the power supply configured to selectively supply electrical power to the sanitization compartment,
   wherein the power supply is configured to receive user input and, in response to the user input, transmit the electrical power to the sanitization compartment.
2. The portable medical system of paragraph 1, wherein the sanitization compartment is configured to transmit UV light in response to receiving the electrical power.
3. The portable medical system of paragraph 1, wherein the sanitization compartment is configured to release a sanitization agent selected from the group consisting of ozone gas, heated air, ethylene oxide, microwave energy, hydrogen peroxide vapor, hydrogen peroxide plasma, and formaldehyde gas.
4. The portable medical system of paragraph 1, wherein the sterilization compartment is removably disposed within the portable medical container.
5. The portable medical system of paragraph 4, wherein the portable medical container further includes a compartment selected from the group consisting of a fluid delivery compartment, an electrosurgical generator, a removable power supply, and an air recirculation system compartment.
6. The portable medical system of paragraph 5, wherein the compartment is removable.
7. The portable medical system of paragraph 1, further comprising a shroud having at least one opening configured have an air connection hose coupled thereto to facilitate air circulation thereacross.
8. The portable medical system of paragraph 7, further comprising a console configured to be in electrical communication with the power supply and a remote console.
9. The portable medical system of paragraph 1, further comprising at least one medical device selected from the group consisting of an imaging device, an electrically powered surgical instrument, and an electrosurgical instrument.
10. The portable medical system of paragraph 1, wherein the power supply is configured to receive electrical power from an external power source and, in response to receiving the electrical power, store the electrical power in on or more batteries disposed in the power supply.
11. The portable medical system of paragraph 8, wherein the console is configured to establish wireless communication with a remote console.
12. The portable medical system 11, wherein, when wireless communication is established between the console and the remote console,
   the console is configured to:
   receive user input at the console; and
   transmit the user input to the remote console, and
   in response to transmitting the user input, receive second user input at the remote console.
13. The portable medical system of paragraph 11, wherein, when wireless communication is established between the console and the remote console, the console is further configured to display the second user input on a display of the console.
14. A method of monitoring a telemedical procedure, the method comprising:
   deploying a console at a remote surgical theater located in a first location;
   establishing electrical communication with a second console in a second location;
   inputting user input into the console at the first location to be transmitted to a clinician interacting with the second console at the second location;
   transmitting the user input from the console at the first location to the console at the second location;
   receiving at the console at the first location, in response to transmitting the user input, second user input received at the second console; and
   displaying the second user input on a display of the console at the first location.
15. The method of paragraph 14, further comprising compressing the user input received at the console at the first location prior to the transmitting.
16. The method of paragraph 15, further comprising compressing the second user input received at the second console prior to the receiving.
17. The method of paragraph 15, further comprising:
   receiving, at the console at the first location, instructions to control a surgical device in electrical communication with the console; and
   transmitting control signals from the console based on the instructions to control the surgical device.

## Claims

1. A portable medical system for deploying a remote surgical theater, the portable medical system comprising:
a portable medical container transitionable between a closed position and an open position;
a sanitization compartment disposed within the portable medical container and transitionable between a closed position and an open position; and
a power supply disposed within the portable medical container, the power supply configured to selectively supply electrical power to the sanitization compartment,
wherein the power supply is configured to receive user input and, in response to the user input, transmit the electrical power to the sanitization compartment.

2. The portable medical system of claim 1, wherein the sanitization compartment is configured to transmit UV light in response to receiving the electrical power.

3. The portable medical system of claim 1 or claim 2, wherein the sanitization compartment is configured to release a sanitization agent selected from the group consisting of ozone gas, heated air, ethylene oxide, microwave energy, hydrogen peroxide vapor, hydrogen peroxide plasma, and formaldehyde gas.

4. The portable medical system of any preceding claim, wherein the sterilization compartment is removably disposed within the portable medical container; preferably wherein the portable medical container further includes a compartment selected from the group consisting of a fluid delivery compartment, an electrosurgical generator, a removable power supply, and an air recirculation system compartment.

5. The portable medical system of claim 4, wherein the compartment is removable.

6. The portable medical system of any preceding claim, further comprising a shroud having at least one opening configured have an air connection hose coupled thereto to facilitate air circulation thereacross; preferably further comprising a console configured to be in electrical communication with the power supply and a remote console.

7. The portable medical system of any preceding claim, further comprising at least one medical device selected from the group consisting of an imaging device, an electrically powered surgical instrument, and an electrosurgical instrument.

8. The portable medical system of any preceding claim, wherein the power supply is configured to receive electrical power from an external power source and, in response to receiving the electrical power, store the electrical power in on or more batteries disposed in the power supply.

9. The portable medical system of any of claims 6 to 8, wherein the console is configured to establish wireless communication with a remote console; preferably wherein, when wireless communication is established between the console and the remote console,
the console is configured to:
receive user input at the console; and
transmit the user input to the remote console, and
in response to transmitting the user input, receive second user input at the remote console.

10. The portable medical system of claim 9, wherein, when wireless communication is established between the console and the remote console, the console is further configured to display the second user input on a display of the console.

11. A method of monitoring a telemedical procedure, the method comprising:
deploying a console at a remote surgical theater located in a first location;
establishing electrical communication with a second console in a second location;
inputting user input into the console at the first location to be transmitted to a clinician interacting with the second console at the second location;
transmitting the user input from the console at the first location to the console at the second location;
receiving at the console at the first location, in response to transmitting the user input, second user input received at the second console; and
displaying the second user input on a display of the console at the first location.

12. The method of claim 11, further comprising compressing the user input received at the console at the first location prior to the transmitting.

13. The method of claim 11 or claim 12, further comprising compressing the second user input received at the second console prior to the receiving.

14. The method of any of claims 11 to 13, further comprising:
receiving, at the console at the first location, instructions to control a surgical device in electrical communication with the console; and
transmitting control signals from the console based on the instructions to control the surgical device.
